# EUROPÄISCHE PATENTANMELDUNG

(11) **EP 1 468 699 A1**
(43) Veröffentlichungstag der Anmeldung: **20.10.2004**
(21) Anmeldenummer: 03008612.8
(22) Anmeldetag: 15.04.2003
(51) Int. Cl.: A61L 2/00, A61L 2/16, A61L 2/18, A61K 31/205

(54) **Mycobakterizides Desinfektionsmittel**

(71) Anmelder: Ecolab Inc., St. Paul, MN 55102-1390 (US)
(72) Erfinder: Meyer, Bernhard, Dr., 40822 Mettmann (DE); Decker, Michael, 42697 Solingen (DE)
(74) Vertreter: Sternagel, Fleischer, Godemeyer & Partner Patentanwälte

(57) **Zusammenfassung**

Es wird die Verwendung von wässerigen Mitteln, die bestimmte Kombinationen aus Alkohol und Amphotensid enthalten, zur Abtötung von Mycobakterien vorgestellt.

## Beschreibung

Die vorliegende Erfindung betrifft die Verwendung von wässerigen Mitteln, die bestimmte Kombinationen aus Alkohol und Amphotensid enthalten, zur Abtötung von Mycobakterien.

Bei der Formulierung von Desinfektionsmitteln wird eine gleichmäßige Wirksamkeit gegen möglichst viele verschiedenen Keime angestrebt. Wie auch schon in der DE 3117792 ausführlich dargelegt, sind ein besonderes Problem dabei die Mycobakterien, die sich mit ihrer Wachshülle gut gegen widrige äußere Einflüsse schützen können. Diese Schutzhülle verhindert zum Beispiel eine Abtötung durch quaternäre Ammoniumverbindungen und/oder mikrobizide Amphotensiden und erzwingt eine Erhöhung der Konzentration von Aldehyden, wenn man gegen Mycobakterien wirksame Desinfektionsmittel erhalten will. Gut wirksam gegen Mycobaktenen sind zum Beispiel Phenol und seine Derivate. Diese werden aber wegen ihres Geruches und wegen schlechter Verträglichkeit mit bestimmten Materialien wie zum Beispiel mit Kunststoffen und Gummi nicht mehr eingesetzt.

Seit mehreren Jahren werden bestimmte Fettaminverbindungen als Wirkstoffe gegen Mycobakterien eingesetzt. Die Produkte, die auf der Basis von Aminderivaten auf dem Markt sind, enthalten neben den Aminen noch quaternäre Ammoniumverbindungen. Eine derartige Kombination von Aminen und quaternären Ammoniumverbindungen ist nur bei hohen pH-Werten gegen Mycobakterien wirksam.

Bei der Desinfektion von medizinischen Produkten wie chirurgischen Instrumenten oder Endoskopen werden in letzter Zeit vermehrt Formulierungen aus quaternären Ammoniumverbindungen und Aldehyden oder sogar aldehydfreie Formulierungen auf Basis von quaternären Ammoniumverbindungen und/oder Amphotensiden eingesetzt. Diese Produkte haben alle den Nachteil einer Wirkungsschwäche oder sogar einer Wirkungslücke gegenüber Mycobakterien.

In letzter Zeit ist versucht worden, die Eigenschaften phenolischer Verbindungen mit dem guten Image der quaternären Ammoniumverbindungen zu kombinieren. Es wurden Produkte auf den Markt gebracht, die neben mindestens einer quaternären Ammoniumverbindung eine stickstoffhaltige Base und Phenoxyethanol oder Phenoxypropanol enthalten. Diese Produkte zeigen eine gute Hemmwirkung gegenüber Mycobakterien, wie sich aus der Druckschrift DE-PS 40 05 784 ergibt. Ob diese Kombinationen Mycobakterien auch abtöten, also als Desinfektionsmittel wirken, ist in der Fachwelt umstritten. Unbestritten ist aber die Tatsache, dass die Phenoxyethanole oder Phenoxypropanole Fußbodenbeschichtungen anlösen und aufweichen können und so die Neuverschmutzung von den Böden beschleunigen und die Lebensdauer der teuren Grundbeschichtungen der Böden verringern können.

Ein weiterer Versuch, gegen Mycobakterien wirksame Desinfektionsmittel bereitzustellen, war die Verwendung von Ketonen, wie zum Beispiel Pentanon, und höheren Alkoholen, wie zum Beispiel Butanol oder Hexanol, in Kombination mit weiteren, als desinfizierend wirkende Mittel bekannten Komponenten. In Kombination mit Aldehyden verbessern diese Verbindungen die Wirksamkeit gegen Mycobakterien. Jedoch lassen der starke Geruch und die hohe Toxizität eine Verwendung in Desinfektionsmitteln nicht geraten erscheinen.

Aufgabe der vorliegenden Erfindung war es, Desinfektionsmittelkonzentrate mit abtötender Wirkung gegen ein breites Spektrum von Keimen und insbesondere Mycobakterien bereitzustellen, die eine gute Transport- und Lagerstabilität aufweisen und sich vom Anwender problemlos, bei guter Materialverträglichkeit auch gegenüber Kunststoffen, Gummi und anderen üblichen Materialien einsetzen lassen.

Eine ähnliche Aufgabenstellung hatte sich bereits die DE 3117792 gestellt. Dabei wurde gefunden, dass Terpene mit den verschiedensten desinfizierend wirkenden Komponenten wie quaternären Ammoniumverbindungen, Aldehyden, Glycolen und Aminen kombiniert werden können und dass es mit aus derartigen terpenhaltigen Desinfektionsmittel-Konzentraten hergestellten Gebrauchsverdünnungen möglich ist, die Nachteile der bisherigen Desinfektionsmittel zu vermeiden. Als überraschend wurde in der DE 3117792 auch dargestellt, dass die Wirksamkeit der Mittel bei Zusatz bereits geringer Terpen-Mengen verbessert werden konnte. Dies zeigte sich auch in einer verbesserten Wirkung gegenüber Mycobakterien.

Daneben waren auch noch weitere Vorteile festgestellt worden, wie die Tatsache, daß terpenhaltige Desinfektionsmittel- Lösungen Bodenbeschichtungen nicht anlösen. Weiter wurde in der DE 31 17 792 gesagt, dass die Terpene selbst den aus den Konzentraten hergestellten Gebrauchsverdünnungen einen angenehmen Geruch verleihen.

Dementsprechend betrifft die DE 31 17 792 Desinfektionsmittel-Konzentrate, die neben an sich bekannten, desinfizierend wirkenden Verbindungen und üblichen Hilfsstoffen sowie Wasser ein oder mehrere Terpene in Konzentrationen von 0,1 bis 50 % enthalten, bezogen auf das Gesamtgewicht des Desinfektionsmittel-Konzentrats.

Unter dem Begriff "Terpene" versteht die DE 3117792 sowohl Terpen-Kohlenwasserstoffe als auch Terpenoide, d. h. Terpen-Alkohole, Terpen-Aldehyde, Terpen-Ketone und Terpen-Ester. Letztere können als Einzelverbindungen oder auch in Mischungen miteinander verwendet werden.

Bevorzugte Terpene gem. der DE 31 17 792 sind Monoterpene, Sesquiterpene, Diterpene, Sesterpene, Triterpene und Tetraterpene. Grundsätzlich sind jedoch auch Polyterpene verwendbar.

Als vorteilhaft stellt die DE 31 17 792 beispielsweise den Einsatz von Terpen-Mischungen dar, die im Handel unter den Bezeichnungen "Orangenterpen" und "Citrusterpen" vertrieben werden und die natürlich in Orangen bzw. in Citrusfrüchten enthaltene Terpene wie beispielsweise Citronellol, Geranial und Neral in für den Einsatz brauchbaren Mischungen enthalten.

Die DE 31 177 92 beschreibt in einer Ausführungsform die Anwendung der erfindungsgemäß zu verwendenden Mittel für die Flächendesinfektion und die Instrumentendesinfektion, vorzugsweise bei Auftreten von Mycobakterien, insbesondere von Tuberculoseerregern.

Die DE 31 17 792 weist jedoch den Nachteil auf, dass nur durch Einsatz von Terpenen eine hinreichende Abtötung von Mycobakterien erreicht wird.

Bekanntermaßen weisen jedoch Terpene aus anwendungstechnischer Sicht eine Reihe von Nachteilen auf.

So weisen sie beispielsweise einen von vielen Anwendern als durchdringend und storend empfundenen Geruch auf. Außerdem sind viele Terpene als sensibilisierend bekannt und können daher Allergien auslösen. Um eine breite Zielgruppe anzusprechen ist es schon aus diesen Gründen nicht ratsam auf Terpen-haltige Produkte zurückzugreifen.

Für denjenigen, der also bereits ohne den nachteiligen Einsatz von Terpenen eine sehr gute Wirkung gegenüber Mycobakterien erreichen will liefert der Stand der Technik keine Hinweise, wie er die weiter oben gestellte Aufgabe lösen kann.

Dementsprechend war es eine weitere Aufgabe der vorliegenden Erfindung, den Effekt der abtötenden Wirkung insbesondere gegenüber Mycobakterien bereits ohne den Einsatz von Terpenen zu erreichen.

Diese Aufgabe wurde überraschenderweise gelöst durch die Verwendung eines wässerigen Mittels, enthaltend bezogen auf das gesamte Desinfektionsmittel
a) 3 bis 30 Gew.-%, vorzugsweise zwischen 5 und 25 Gew.-% und insbesondere zwischen 10 und 20 Gew.-% wenigstens eines Alkohols ausgewählt aus der Gruppe der einwertigen niedermolekularen Alkohole gemäß Formel (I), 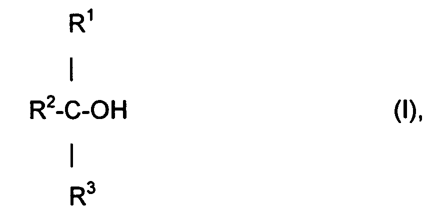 in der R¹, R² und R³ unabhängig voneinander H-Atome oder Alkylreste mit jeweils 1 bis 3 C-Atomen umfasst, wobei die Gesamtzahl der C-Atome nicht größer als 6 ist und
b) 0,01 bis 3 Gew.-%, vorzugsweise 0,05 bis 1 Gew.-%, wenigstens eines amphoteren Tensids
   zur Abtötung von Mykobakterien. Dabei sei an dieser Stelle erwähnt, daß das erfindungsgemäß zu verwendende Mittel üblicherweise in unverdünnter Form verwendet wird.

Das genannte in dem erfindungsgemäß zu verwendenden Mittel enthaltene amphotere Tensid ist vorzugsweise ausgewählt aus der Gruppe der Betaine, insbesondere aus den Verbindungen der Formel (II) 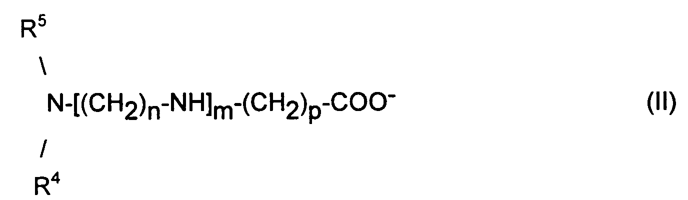 und/oder den Verbindungen der Formel (III) 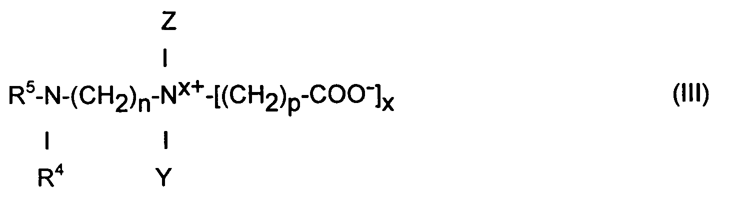 wobei
- R⁵: ein Rest R, R-O oder R-CO
- R: Wasserstoff, ein substituierter oder unsubstituierter, linearer oder verzweigter, gesättigter oder ein-, zwei- oder dreifach ungesättigter Alkylrest mit 8 bis 20 C-Atomen, wobei die Substituenten ausgewählt sind aus Amino-, Imino-, Hydroxy- und Carboxygruppen,
- R⁴: Wasserstoff, eine Alkylgruppe mit 3 bis 12 C-Atomen, eine -(CH₂-CH₂-O)₁₋₃- H - Gruppe, eine Gruppe -[(CH₂)ₙ-NH]ₗ-(CH₂)ₚ-COO⁻ oder eine Gruppe -[(CH₂)ₙ-NH]ₗ-H,
- Y: ein Substituent CH₃ oder -(CH₂)ₚ-O-X oder -(CH₂)ₘ-COOM oder ein substituierter oder unsubstituierter, linearer oder verzweigter, gesättigter oder ein-, zwei- oder dreifach ungesättigter Alkylrest mit 8 bis 20 C-Atomen,
- Z: unabhängig von Y ein Substituent CH₃ oder -(CH₂)ₚ-O-X oder -(CH₂)ₘ-COOM oder ein substituierter oder unsubstituierter, linearer oder verzweigter, gesättigter oder ein-, zwei- oder dreifach ungesättigter Alkylrest mit 8 bis 20 C-Atomen,
- X: Wasserstoff oder eine Gruppe -(CH₂)ₘ-COOM,
- M: unabhängig voneinander Wasserstoff, ein Ammonium-, Alkylammonium- oder Alkalimetallkation,
- n: unabhängig voneinander 1, 2 oder 3,
- m: unabhängig voneinander 0, 1, 2, 3 oder 4
- p: unabhängig voneinander 1, 2 oder 3
- l: 1 oder 2
- x: 0 oder 1
bedeuten.

Beispielsweise sind mögliche Amphotere die Verbindungen Dimethyl-C8-C18-acylamido-propyl-acetobetain (Dehyton® K von Cognis) und/oder Glycin-N-(3-Aminopropyl)-C10-C16-Alkylderivate (Amphionic® SFB von Rhodia) und/oder die amphoteren Tenside der Fa. Goldschmidt, die unter dem Markennamen TEGO® vermarktet werden.

Bevorzugt ist eine Ausführungsform des genannten amphoteren Tensids gemäß der allgemeinen Formel (II), in der n = 2 oder 3 oder p = 1 oder m = 2 oder R⁴ = H oder R⁵ ein gesättigter Alkylrest mit 10 bis 16 C-Atomen sind, insbesondere n = 3 und p = 1 und m = 2 und R⁴ = H und R⁵ ein gesättigter Alkylrest mit 10 bis 16 C-Atomen sind.

Eine ebenfalls bevorzugte Ausführungsform des genannten amphoteren Tensids gemäß der allgemeinen Formel (II) liegt vor, wenn n = 2 oder 3 oder p = 1 oder m = 0 oder R⁴ = [(CH₂)ₙ-NH]ₗ-H mit l =1 oder R⁵ ein gesättigter Alkylrest mit 10 bis 16 C-Atomen sind, vorzugsweise n = 3 und p = 1 und m = 0 und R⁴ = [(CH₂)ₙ-NH]ₗ-H mit l =1 und R⁵ ein gesättigter Alkylrest mit 10 bis 16 C-Atomen sind.

Eine weitere bevorzugte Ausführungsform des genannten amphoteren Tensids gemäß der allgemeinen Formel (III) besteht dann, wenn n = 3 oder m= 0 oder x = 0 oder Y = H oder Z = -(CH₂)ₘ-COOM oder R⁴ = H oder R⁵ ein gesättigter Alkylrest mit 10 bis 16 C-Atomen, vorzugsweise n = 3 und m= 0 und x = 0 und Y = H und Z = -(CH₂)ₘ-COOM und R⁴ = H und R⁵ ein gesättigter Alkylrest mit 10 bis 16 C-Atomen sind.

Bevorzugt ist ebenfalls eine Ausführungsform des genannten amphoteren Tensids gemäß der allgemeinen Formel (III), in der n = 3 oder x = 1 oder p = 1 oder Y = CH₃ oder Z = CH₃ oder R⁴ = H oder R⁵ ein gesättigter Alkylrest mit 8 bis 18 C-Atomen, vorzugsweise n = 3 und x = 1 und p = 1 und Y = CH₃ und Z = CH₃ und R⁴ = H und R⁵ ein gesättigter Alkylrest mit 8 bis 18 C-Atomen sind.

Dabei liegt eine weitere bevorzugte Ausführungsform des genannten amphoteren Tensids gemäß der allgemeinen Formel (III) vor, wenn n = 3 und p = 1 und x = 0 und Y = H und Z = -(CH₂)ₘ-COOM und R⁴ = H und R⁵ ein gesättigter Alkylrest mit 10 bis 16 C-Atomen sind.

In einer bevorzugten Ausführungsform ist der in dem erfindungsgemäß zu verwendenden Mittel enthaltene Alkohol gemäß Formel (I) ausgewählt aus Ethanol, 1-Propanol und 2-Propanol.

Das erfindungsgemäß zu verwendende Mittel wird außerdem bevorzugt zur Desinfektion von Oberflächen, insbesondere harten Oberflächen eingesetzt, wobei es besonders bevorzugt ist, das genannte Mittel in unverdünnter Form auf die genannten Oberflächen aufzubringen und gewünschtenfalls nach ausreichender Einwirkzeit durch Abspülen mit Wasser zu entfernen oder z.B. mittels eines Papiertuches abzuwischen.

Die Vorteile des erfindungsgemäß zu verwendenden Mittels kommen insbesondere dann zum Tragen, wenn es zur Behandlung von Oberflächen, hauptsächlich von harten Oberflächen, im medizinischen Bereich, in der Lebensmittel herstellenden und/oder verarbeitenden Industrie, im Hotelgewerbe, in öffentlichen Gebäuden und Institutionen mit dem genannten Desinfektionsmittel behandelt werden. Als harte Oberflächen sind beispielsweise zu verstehen medizinische Instrumente, Geräte oder sonstige medizinische Ausrüstungsgegenstände, Betten, Wände, Böden, Fenster, Maschinen, Tanks, Rohrleitungen, Transportbänder, Durchlaufwaschmaschinen, Flaschen.

Darüber hinaus sind alle Flächen, die einen direkten Kontakt zum menschlichen Korper haben, z.B. Flächen in Sanitärbereichen, Wärmebänke in öffentlichen Bädern oder Sonnenbänke in Sonnenstudios, als zu behandelnde Oberflächen zu verstehen.

Die Erfindung betrifft dabei insbesondere die Anwendung der erfindungsgemäß zu verwendenden Mittel für die Oberflächendesinfektion, vorzugsweise bei Auftreten von Mycobakterien, insbesondere von Tuberkuloseerregern, aber auch dann, wenn das Auftreten der genannten Bakterien vorausschauend vermieden werden soll.

Die erfindungsgemäß zu verwendenden Mittel können mit weiteren an sich bekannten, desinfizierend wirkenden Verbindungen kombiniert werden.

Als solche kommen bevorzugt Aldehyde in Frage. Dabei können aus dieser Stoffklasse eine Einzelverbindung oder Mischungen mehrerer Verbindungen zum Einsatz kommen. Besonders bevorzugte Beispiele sind Glutardialdehyd, Formaldehyd, Ethylhexanal und Succindialdehyd.

Als weitere an sich bekannte, desinfizierend wirkende Verbindungen, die mit dem erfindungsgemäß zu verwendenden Mittel kombiniert werden können, kommen außerdem quaternäre Ammoniumverbindungen in Frage. Auch aus dieser Stoffklasse kann eine Einzelverbindung oder Mischungen mehrerer Verbindungen zum Einsatz kommen. Besonders bevorzugte Beispiele sind Benzalkoniumchlorid, Didecyldimethylammoniumchlorid, Dioctyldimethylam-moniumchlorid, Mecetroniumetilsulfat, Didecylmethyloxethylammoniumpropionat, Cetrimid, Chlorhexidin und Decylisononyldimethyl-ammoniumchlorid. In der Praxis können im Handel erhältlich Einzelverbindungen oder Mischungen der genannten oder anderer mikrobizider quaternärer Ammoniumverbindungen verwendet werden. Ein Beispiel hierfür ist das unter der Bezeichnung Bardap 114 von der Firma Lonza vertriebene Produkt.

Weitere mögliche Kombinationspartner für das erfindungsgemäß zu verwendenden Mittel sind außerdem die als bekannte, desinfizierend wirkende Verbindungen zu bezeichnenden Amine und/oder Aminsalze. Auch aus dieser Stoffklasse kann eine Einzelverbindung oder Mischungen mehrerer Verbindungen zum Einsatz kommen. Besonders bevorzugte Beispiele sind Cocosdimethylamin, Cocosdiethylentriamin, Cocosdiethanolamin und ein tertiäres Alkylamin. In der Praxis können im Handel erhältliche Einzelverbindungen oder Mischungen der genannten Amine und/oder Aminsalze verwendet werden. Ein Beispiel hierfür ist das unter der Bezeichnung LONZABAC 12 von der Firma Lonza AG vertriebene Produkt.

Die Erfindung wird nachfolgend anhand der Beispiele näher erläutert, ohne jedoch auf die in den Beispielen beschriebenen Ausführungsformen beschränkt zu sein.

### Beispiele:

### 1. Herstellung von erfindungsgemäßen Beispielen E1, E2 und E3 sowie Vergleichsbeispielen V1, V2, V3

Durch einfaches Zusammenfügen verschiedener Einzelbestandteile, vorzugsweise unter Rühren, wurden die erfindungsgemäßen Mittel E1, E2 und E3 sowie die Vergleichsbeispiele V1, V2 und V3 gemäß Tabelle 1 hergestellt.

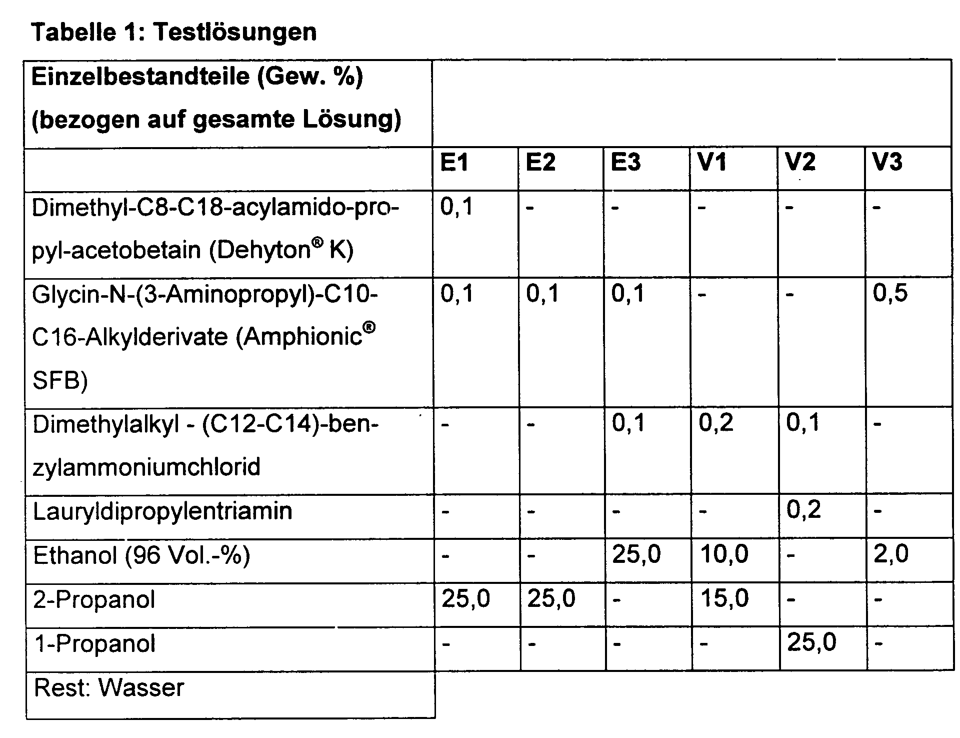

### 2. Prüfung der Materialverträglichkeit der erfindungsgemäßen Mittel E1, E2 und E3 sowie der Vergleichsformulierungen V1, V2, V3 im Biegestreifentest mit Plexiglas in Anlehnung an DIN 53449

Zur Überprüfung der Materialverträglichkeit der erfindungsgemäßen Mittel E1, E2 und E3 sowie der Vergleichsformulierungen V1, V2, V3 wurde der Biegestreifentest mit Plexiglas in Anlehnung an DIN 53449 durchgeführt.
Bei der Bewertung der Ergebnisse wurde visuell beurteilt, wie stark die Rissbildung ausgeprägt war und es wurden entsprechende Bewertungen von 1 (keine Risse) bis 6 (sehr starke Rissbildung) vergeben.

Die internen Anforderungen gelten als erfüllt, wenn das Bewertungkriterium bei diesem Test in einem Bereich um 1 oder 2 liegt. Dies trifft zum einen zu für die erfindungsgemäßen Mittel E1, E2 und E3 (Tabelle 2). Wie aus Tabelle 2 hervorgeht, erreichen die Vergleichsformulierungen V1 und V2 diese Anforderungen nicht. Sie liegen beide in einem Bereich um 4. Hingegen weist die Vergleichsformulierung V3 eine ganz gute Verträglichkeit in diesem Test auf.

**Tabelle 2:**

| **Materialverträglichkeit der erfindungsgemäßen Mittel E1, E2 und E3 sowie der Vergleichsformulierungen V1, V2, V3 im Biegestreifentest mit Plexiglas in Anlehnung an DIN 53449** | |
|---|---|
| | **Bewertung** **Kriterien: von 1(keine Risse) bis 6 (starke Rißbildung)** |
| **Prüfpräparat** | |
| Mittel E1 | 1-2 |
| Mittel E2 | 1-2 |
| Mittel E3 | 2 |
| Formulierung V1 | 4 |
| Formulierung V2 | 4 |
| Formulierung V3 | 1-2 |

### 3. Prüfung der Wirksamkeit der erfindungsgemäßen Beispiele E1, E2 und E3 sowie der Vergleichsbeispiele V1, V2, V3 gegenüber Staphylococcus aureus.

Die Prüfungen erfolgten im Fläschentest nach den Richtlinien der Deutschen Gesellschaft für Hygiene und Mikrobiologie (DGHM-Flächentest) gegen den Testkeim Staphylococcus aureus mit den unverdünnten Mitteln E1, E2 und E3 sowie mit den unverdünnten Vergleichsformulierungen V1, V2, V3. Die Ergebnisse sind in Tabelle 3 zusammengefaßt. Es zeigt sich, daß gegen diesen Testorganismus sowohl mit den erfindungsgemäßen Beispielen als auch mit den Vergleichsformulierungen bei 1 Minute Einwirkzeit gute bis sehr gute logarithmische Reduktionsfaktoren erreicht wurden. Aber wie schon aus Tabelle 2 zu entnehmen war, weisen die Vergleichsformulierungen gravierende Schwächen hinsichtlich der Materialverträglichkeit auf.

**Tabelle 3:**

| **Wirksamkeit der erfindungsgemäßen Mittel E1, E2 und E3 sowie der Vergleichsformulierungen V1, V2, V3 gegen das grampositive Bakterium Staphylococcus aureus im DGHM-Flächentest** | |
|---|---|
| | **Keimreduktionsfaktor nach 1 Min. Einwirkzeit** **(log-Stufen)** |
| **Prüfpräparat** | |
| Mittel E1 | > 6,93 |
| Mittel E2 | >6,93 |
| Mittel E3 | >6,93 |
| Formulierung V1 | >6,93 |
| Formulierung V2 | >6,93 |
| Formulierung V3 | 3,92 |

### 4. Prüfung der Wirksamkeit der erfindungsgemäßen Beispiele E1, E2 und E3 sowie der Vergleichsbeispiele V1, V2, V3 gegenüber Mycobacterium terrae.

Die Prüfungen zur bakteriziden Wirksamkeit gegen Mycobacterium terrae erfolgten im quantitativen Suspensionstest nach den Richtlinien der Deutschen Gesellschaft für Hygiene und Mikrobiologie gegen den Testkeim Mycobacterium terrae mit den unverdünnten erfindungsgemäßen Mitteln E1, E2 und E3 sowie mit den unverdünnten Vergleichsformulierungen V1, V2 und V3. Die Ergebnisse sind in Tabelle 4 zusammengefaßt. Es zeigt sich, daß mit den erfindungsgemäßen Beispielen bei 5 Minuten Einwirkzeit ohne Belastung und bei 15 Minuten Einwirkzeit mit Belastung sehr günstige logarithmische Reduktionsfaktoren erreicht wurden. Zwar zeigen auch die Vergleichsformulierungen V1 und V2 gute Abtötungswerte. Jedoch waren diese Formulierungen, wie aus Tabelle 2 zu entnehmen ist, aufgrund der schlechten Materialverträglichkeit nicht geeignet. Die in den Beispielen gemäß Tabelle 2 materialverträgliche Vergleichsformulierung V1 weist jedoch, wie aus Tabelle 4 zu entnehmen ist, keine ausreichende Abtötungswirkung gegenüber Mycobacterium terrae auf.

**Tabelle 4:**

| **Wirksamkeit der erfindungsgemäßen Mittel E1, E2 und E3 sowie der Vergleichsformulierungen V1, V2, V3 gegenüber Mycobakterium terrae im quantitativen Suspensionstest der DGHM** | | |
|---|---|---|
| | **Keimreduktionsfaktor** **(log-Stufen)** | |
| **Prüfpräparat** | **Nach 5 Min. Einwirkzeit** | **nach 15 Min. Einwirkzeit (mit hoher Eiweißbelastung)** |
| Mittel E1 | > 6,8 | > 6,65 |
| Mittel E2 | 4,82 | >6,65 |
| Mittel E3 | >6,8 | >6,65 |
| Formulierung V1 | >6,8 | >6,65 |
| Formulierung V2 | >6,8 | >6,65 |
| Formulierung V3 | <1,18 | <1,18 |

## Patentansprüche

1. Verwendung eines wässerigen Mittels, enthaltend, bezogen auf das gesamte Desinfektionsmittel,
a) 3 bis 30 Gew.-% wenigstens eines Alkohols ausgewählt aus der Gruppe der einwertigen niedermolekularen Alkohole gemäß Formel (I), 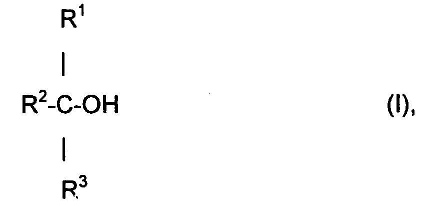 in der R¹, R² und R³ unabhängig voneinander H-Atome oder Alkylreste mit jeweils 1 bis 3 C-Atomen umfasst, wobei die Gesamtzahl der C-Atome nicht größer als 6 ist und
b) 0,01 bis 3 Gew.-%, vorzugsweise 0,05 bis 1 Gew.-%, wenigstens eines amphoteren Tensids zur Abtötung von Mykobakterien.

2. Ausführungsform nach Anspruch 1, **dadurch gekennzeichnet, dass** das genannte Mittel, bezogen auf das gesamte Mittel zwischen 5 und 25 Gew.-%, vorzugsweise zwischen 10 und 20 Gew.-% des genannten Alkohols gem. Formel (I) enthält.

3. Ausführungsform nach einem der Ansprüche 1 oder 2, **dadurch gekennzeichnet, dass** das genannte amphotere Tensid ausgewählt ist aus der Gruppe der Betaine, insbesondere aus den Verbindungen der Formel (II) 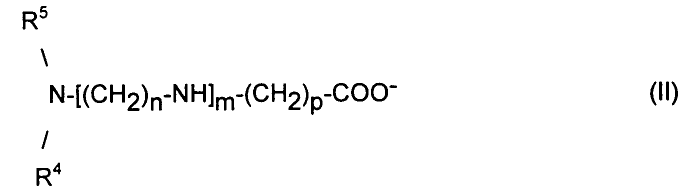 und/oder den Verbindungen der Formel (III) 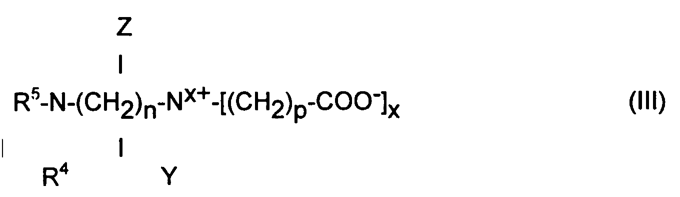 wobei
R⁵ ein Rest R, R-O oder R-CO
R Wasserstoff, ein substituierter oder unsubstituierter, linearer oder verzweigter, gesättigter oder ein-, zwei- oder dreifach ungesättigter Alkylrest mit 8 bis 20 C-Atomen, wobei die Substituenten ausgewählt sind aus Arnino-, Imino-, Hydroxy- und Carboxygruppen,
R⁴ Wasserstoff, eine Alkylgruppe mit 3 bis 12 C-Atomen, eine -(CH₂-CH₂-O)₁₋₃-H - Gruppe, eine Gruppe -[(CH₂)ₙ-NH]ₗ-(CH₂)ₚ-COO⁻ oder eine Gruppe -[(CH₂)ₙ-NH]ₗ-H,
Y ein Substituent CH₃ oder -(CH₂)ₚ O-X oder -(CH₂)ₘ-COOM oder ein substituierter oder unsubstituierter, linearer oder verzweigter, gesättigter oder ein-, zwei- oder dreifach ungesättigter Alkylrest mit 8 bis 20 C-Atomen,
Z unabhängig von Y ein Substituent CH₃ oder -(CH₂)ₚ-O-X oder -(CH₂)ₘ-COOM oder ein substituierter oder unsubstituierter, linearer oder verzweigter, gesättigter oder ein-, zwei- oder dreifach ungesättigter Alkylrest mit 8 bis 20 C-Atomen,
X Wasserstoff oder eine Gruppe -(CH₂)ₘ-COOM,
M unabhängig voneinander Wasserstoff, ein Ammonium-, Alkylammonium- oder Alkalimetallkation,
n unabhängig voneinander 1, 2 oder 3,
m unabhängig voneinander 0, 1, 2, 3 oder 4
p unabhängig voneinander 1, 2 oder 3
l 1 oder 2
x 0 oder 1
bedeuten.
Als Beispiele werden in der Beschreibung dargestellt:
Dimethyl-C8-C18-acylamido-propyl-acetobetain (Dehyton® K)
Glycin-N-(3-Aminopropyl)-C10-C16-Alkylderivate (Amphionic® SFB)
Die TEGO Biozide

4. Ausführungsform nach Anspruch 3, **dadurch gekennzeichnet, dass** in der allgemeinen Formel (III) n = 2 oder 3 oder p = 1 oder m = 2 oder R⁴ = H oder R⁵ ein gesättigter Alkylrest mit 10 bis 16 C-Atomen sind, vorzugsweise n = 3 und p = 1 und m = 2 und R⁴ = H und R⁵ ein gesättigter Alkylrest mit 10 bis 16 C-Atomen sind.

5. Ausführungsform nach Anspruch 3, **dadurch gekennzeichnet, dass** in der allgemeinen Formel (II) n = 2 oder 3 oder p = 1 oder m = 0 oder R⁴ = [(CH₂)ₙ-NH]ₗ-H mit l =1 oder R⁵ ein gesättigter Alkylrest mit 10 bis 16 C-Atomen sind, vorzugsweise n = 3 und p = 1 und m = 0 und R⁴ = [(CH₂)ₙ-NH]ₗ-H mit l =1 und R⁵ ein gesättigter Alkylrest mit 10 bis 16 C-Atomen sind.

6. Ausführungsform nach Anspruch 3, **dadurch gekennzeichnet, dass** in der allgemeinen Formel (III) n = 3 oder m= 0 oder x = 0 oder Y = H oder Z = -(CH₂)ₘ-COOM oder R⁴ = H oder R⁵ ein gesättigter Alkylrest mit 10 bis 16 C-Atomen, vorzugsweise n = 3 und m= 0 und x = 0 und Y = H und Z = -(CH₂)ₘ-COOM und R⁴ = H und R⁵ ein gesättigter Alkylrest mit 10 bis 16 C-Atomen sind.

7. Ausführungsform nach Anspruch 3, **dadurch gekennzeichnet, dass** in der allgemeinen Formel (III) n = 3 oder x = 1 oder p = 1 oder Y = CH₃ oder Z = CH₃ oder R⁴ = H oder R⁵ ein gesättigter Alkylrest mit 8 bis 18 C-Atomen, vorzugsweise n = 3 und x = 1 und p = 1 und Y = CH₃ und Z = CH₃ und R⁴ = H und R⁵ ein gesättigter Alkylrest mit 8 bis 18 C-Atomen sind.

8. Ausführungsform nach Anspruch 3, **dadurch gekennzeichnet, dass** in der allgemeinen Formel (III) n = 3 und p = 1 und x = 0 und Y = H und Z = -(CH₂)ₘ-COOM und R⁴ = H und R⁵ ein gesättigter Alkylrest mit 10 bis 16 C-Atomen sind.

9. Ausführungsform nach Anspruch 8, **dadurch gekennzeichnet, dass** der genannte Alkohol ausgewählt ist aus Ethanol, 1-Propanol und 2-Propanol.

10. Ausführungsform nach einem oder mehreren der Ansprüche 1 bis 9, **dadurch gekennzeichnet, daß** das genannte Mittel zur Desinfektion von Oberflächen, insbesondere harten Oberflächen eingesetzt wird.

11. Ausführungsform nach Anspruch 10, **dadurch gekennzeichnet, daß** das genannte Mittel in unverdünnter Form auf die genannten Oberflächen aufgebracht wird und gewünschtenfalls nach ausreichender Einwirkzeit durch Abspülen mit Wasser wieder entfernt wird.

12. Ausführungsform nach Anspruch 11, **dadurch gekennzeichnet, dass** Oberflächen, insbesondere von Gegenständen, im medizinischen Bereich, in der Lebensmittel herstellenden und/oder verarbeitenden Industrie, im Hotelgewerbe, in öffentlichen Gebäuden und Institutionen mit dem genannten Desinfektionsmittel behandelt werden.
